# EUROPEAN PATENT APPLICATION

(11) **EP 2 305 331 A1**
(43) Date of publication of application: **06.04.2011**
(21) Application number: 09762541.2
(22) Date of filing: 12.06.2009
(51) Int. Cl.: A61M 1/14

(54) **DIALYZATE PREPARATION METHOD AND DIALYZATE PREPARATION DEVICE**

(30) Priority: 13.06.2008 JP 2008154918
(71) Applicant: NIPRO CORPORATION, Osaka-shi Osaka 531-8510 (JP); Shibuya Kogyo Co., Ltd., Kanazawa-shi Ishikawa 920-8681 (JP)
(72) Inventor: UEDA, Mitsutaka, Osaka-shi Osaka 531-8510 (JP); SAWADA, Toshiharu, Kanazawa-shi Ishikawa 920-8681 (JP); FUJIKAWA, Inobu, Kanazawa-shi Ishikawa 920-8681 (JP)
(74) Representative: Naylor, Matthew John
(86) International application number: PCT/JP2009/060735
(87) International publication number: WO 2009/151111

(57) **Abstract**

Powder A dissolving means 1 and powder B dissolving means 1 prepare a solution A and a solution B by dissolving a dialysis powder A and a dialysis powder B, respectively, in purified water. The solution A and the solution B with prescribed concentrations are prepared beforehand so that a dialysate can be obtained by mixing the solution A and the solution B in the prescribed proportions without the addition of purified water. Weighing means 4 of a dialysate preparation device 3 feeds the solution A and the solution B to a reciprocating pump 5 so that the solutions are mixed in the prescribed proportions, and in the reciprocating pump 5 the solution A and the solution B with the set concentrations are mixed in the prescribed proportions, whereby it is possible to prepare the dialysate without adding purified water. Dialysates can be easily prepared.

## Description

### Technical Field

The present invention relates to a dialysate preparation method and a dialysate preparation device and, more particularly, to a dialysate preparation method and a dialysate preparation device which are used in preparing a dialysate by mixing a solution A and a solution B, which are obtained by dissolving a dialysis powder A and a dialysis powder B, separately, in purified water.

### Background Art

As a method of preparing a dialysate used in dialytic treatment, mixing an undiluted liquid A, an undiluted liquid B and purified water in prescribed proportions has hitherto been carried out (Patent Literature 1, Patent Literature 2), and it is known that the above-described undiluted liquid A and undiluted liquid B are obtained by dissolving a dialysis powder A and a dialysis powder B, separately, in purified water (Patent Literature 3).

### Prior art documents

### Patent Literatures

Patent Literature 1: Japanese Patent Publication No. 5-34988
Patent Literature 2: Japanese Patent Laid-Open No. 7-275352
Patent Literature 3: Japanese Patent Laid-Open No. 10-232

### Summary of Invention

### Problems to be solved by the invention

The mixing ratio of the undiluted liquid A, the undiluted liquid B and purified water for preparing a dialysate is, for example, 1:1.26:32.74, and as described in Patent Literature 3, also in the case where the undiluted liquid A and the undiluted liquid B are prepared by dissolving the dialysis powder A and the dialysis powder B in purified water, it is ensured that the undiluted liquid A and the undiluted liquid B are prepared with such dissolved concentrations that a dialysate is obtained by mixing the undiluted liquid A and the undiluted liquid B with the purified water at the above-described mixing ratio.
For this reason, in preparing a dialysate, it is necessary that after the preparation of the undiluted liquids by dissolving the above-described powders in purified water, the undiluted liquids be further diluted with purified water, posing the problem of complicated work.
Also, conventional dialysate preparation devices are provided with two mixing tanks which mix the undiluted liquid A, the undiluted liquid B and purified water, which are separately fed, and a storage tank which houses a dialysate prepared in each of the mixing tanks, each of the mixing tanks alternately feeds the prepared dialysate to the storage tank, and the dialysate is simultaneously supplied from this storage tank to a large number of dialysis devices continuously.
For this reason, it is impossible to reduce the size of the above-described mixing tanks and liquid storage tank, posing the problem that equipment cannot be miniaturized.
In view of such problems, the present invention provides a dialysate preparation method and a dialysate preparation device which enable a dialysate to be easily prepared and permit miniaturization of equipment.

### Means for solving the problems

That is, the dialysate preparation method in the first aspect of the present invention is a dialysate preparation method for preparing a dialysate which involves preparing a solution A and a solution B by dissolving a dialysis powder A and a dialysis powder B, separately, in purified water and mixing the solution A and the solution B. This dialysate preparation method comprises: setting beforehand the concentration of the solution A, the concentration of the solution B and prescribed proportions of the solutions so that the dialysate can be prepared by mixing the solution A and the solution B in the prescribed proportions without the addition of purified water; preparing then a solution A with the set concentration by dissolving the dialysis powder A in purified water and preparing a solution B with the set concentration by dissolving the dialysis powder B in purified water; and further preparing the dialysate by mixing the solution A with the set concentration and the solution B with the set concentration in the prescribed proportions without the addition of purified water.

The dialysate preparation device in the third aspect of the present invention comprises: weighing means which separately weighs a solution A with a set concentration and a solution B with a set concentration, which are set so that a dialysate can be obtained by mixing the solutions in prescribed proportion without the addition of purified water; mixing means which prepares a dialysate by mixing the solution A and the solution B, which are weighed by the weighing means; and liquid feeding means which feeds the solution A and the solution B from the weighing means to the mixing means. In this dialysate preparation device, the weighing means weighs the solution A and the solution B in the same number of times so that the prescribed proportions are obtained in the mixing means, and the solution A and the solution B are fed by the liquid feeding means to the mixing means.

### Advantageous Effects of Invention

According to the above-described invention, because the concentration of the solution A, the concentration of the solution B and prescribed proportions of the solutions are set beforehand so that the dialysate can be prepared by mixing the solution A and the solution B in the prescribed proportions without the addition of purified water, a dialysate is obtained by mixing only the solution A and the solution B and it is possible to prepare a dialysate easily.
In the above-described dialysate preparation device in the third aspect, because the mixing device can prepare a dialysate simply by mixing the solution A and the solution B and, it is possible to immediately supply the dialysate to dialysis devices and two mixing tanks and a large storage tank as in conventional dialysate preparation devices become, unnecessary. Therefore, it becomes possible to miniaturize dialysate preparation devices.

### Brief Description of Drawings

[Figure 1] Figure 1 is a block diagram of a dialysate preparation device in Embodiment 1.
[Figure 2] Figure 2 is a block diagram of the dialysate preparation device in a condition occurring after a lapse of a prescribed time from the condition of Figure 1.
[Figure 3] Figure 3 is a block diagram of the dialysate preparation device in a condition occurring after a lapse of a prescribed time from the condition of Figure 2.
[Figure 4] Figure 4 is a block diagram of the dialysate preparation device in a condition occurring after a lapse of a prescribed time from the condition of Figure 3.
[Figure 5] Figure 5 is a block diagram of a dialysate preparation device in Embodiment 2.
[Figure 6] Figure 6 is a block diagram of the dialysate preparation device in a condition after a lapse of a prescribed time from the condition of Figure 5.
[Figure 7] Figure 7 is a block diagram of a dialysate preparation device in Embodiment 3.

### Mode for carrying out the invention

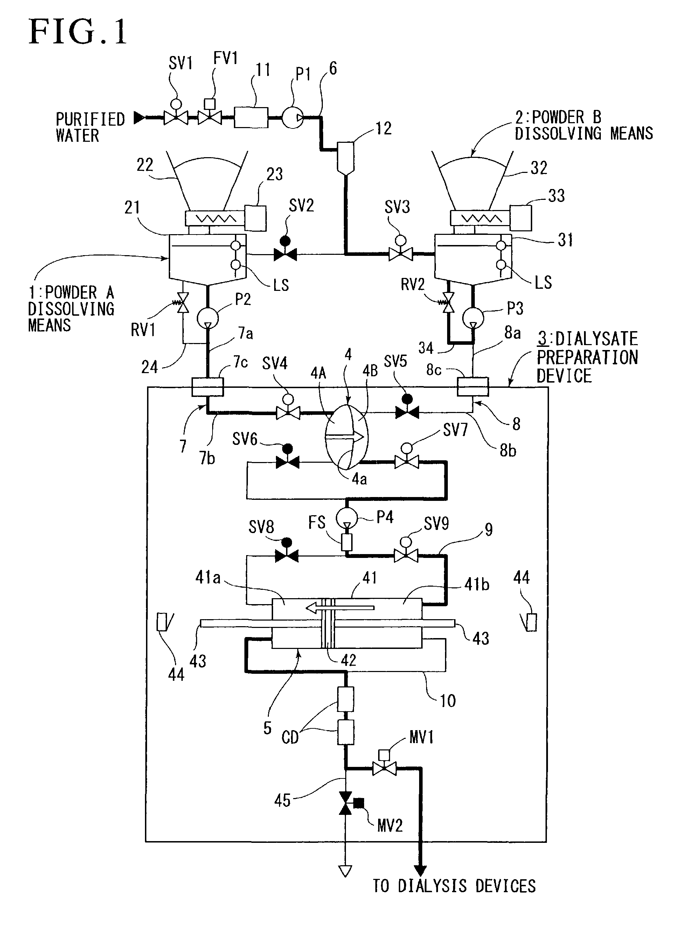

Hereinafter, the first embodiment will be described. Figures 1 to 4 show powder A dissolving means 1 which prepares a solution A by dissolving a dialysis powder A in purified water, powder B dissolving means 2 which prepares a solution B by dissolving a dialysis powder B in purified water, and a dialysate preparation device 3 which prepares a dialysate by mixing the solution A and the solution B, and it is ensured that they are controlled by each control means not shown in the figures.
The above-described dialysate preparation device 3 is provided with weighing means 4 which weighs the solution A and the solution B, which are supplied from the powder A dissolving means 1 and the powder B dissolving means 2, respectively, liquid feeding means which feeds the weighed solution A and solution B, and a reciprocating pump 5 which prepares a dialysate by mixing the fed solution A and solution B, and supplies this dialysate to dialysis devices not shown in the figures.
A purified water passage 6 which supplies purified water is connected to the above-described powder A dissolving means 1 and powder B dissolving means 2. A solution A passage 7 is arranged between the powder A dissolving means 1 and the weighing means 4 of the dialysate preparation device 3, a solution B passage 8 is arranged between the powder B dissolving means 2 and the weighing means 4 of the dialysate preparation device 3, a solution A /solution B passage 9 is arranged between the weighing means 4 and the reciprocating pump 5, and a dialysate passage 10 is arranged between the reciprocating pump 5 and the dialysis devices.
The solution A passage 7 and the solution B passage 8 are composed of discharge passages 7a, 8a, which are on the powder A and powder B dissolving means 1, 2 sides, respectively, inlet passages 7b, 8b, which are on the dialysate preparation device 3 side, and connections 7c, 8c which connect them. It is ensured that the above-described powder A dissolving means 1 and powder B dissolving means 2 are connected by the above-described connections 7c, 8c to the above-described dialysate preparation device 3.

Purified water from purified water supply means, which is not shown, flows into the above-described purified water passage 6, which branches off midway and is connected to the above-described powder A dissolving means 1 and powder B dissolving means 2.
The purified water passage 6 is provided with, in order from the upstream side, a first on-off valve SV1, a flow regulating valve FV1, a heater 11, a first liquid feeding pump P1 and a deaeration tank 12. The branched purified water passage 6 on the powder A dissolving means 1 side is provided with a second on-off valve SV2, and the branched purified water passage 6 on the powder B dissolving means 2 side is provided with a third on-off valve SV3.
The above-described flow regulating valve FV1 causes purified water to flow at a prescribed flow rate, the above-described heater 11 causes the temperature of purified water to rise to a prescribed level, and it is ensured that in deaeration tank 12, air is removed from this purified water.

The above-described powder A dissolving means 1 is provided with a solution A tank 21 to which the above-described purified water passage 6 is connected, a powder A hopper 22 which stores a dialysis powder A, and a dialysis powder A feeder 23 which feeds a dialysis powder A into the solution A tank 21.
The above-described solution A passage 7 is such that the discharge passage 7a is provided with a second liquid feeding pump P2 which feeds the liquid of the solution A tank 21, a solution A circulation passage 24 which branches off from the discharge passage 7a in a position on the downstream side of the second liquid feeding pump P2 and is connected to the solution A tank 21, and a first relief valve RV1 provided in the solution A circulation passage 24, and that the inlet passage 7b is provided with a fourth on-off valve SV4.
The purified water flowing through the above-described purified water passage 6 is supplied to the above-described solution A tank 21 at a prescribed flow rate via the above-described second on-off valve SV2, and the above-described powder A feeder 23 supplies the dialysis powder A in the powder A hopper 22 to this purified water in a prescribed proportion, whereby the dialysis powder A is dissolved.
The solution A tank 21 is provided with a liquid level sensor LS which detects the liquid level of the solution A, and when the liquid level becomes too high or too low, the preparation of the solution A is temporarily stopped and the above-described control means is adapted to output a prescribed alarm.
The above-described second liquid feeding pump P2 is adapted to feed the liquid in the solution A tank 21, and it is ensured that when the fourth on-off valve SV4 is in a closed condition, the fed liquid circulates to the solution A tank 21 after circulation through the solution A circulation passage 24.
On that occasion, because the backward flow of the above-described liquid is prevented by the above-described first relief valve RV1 and furthermore the liquid in the solution A tank 21 is stirred by the circulation of the liquid, it is ensured that the dialysis powder A becomes dissolved in purified water and the solution A is prepared.

The above-described powder B dissolving means 2 is provided with a solution B tank 31 to which the above-described purified water passage 6 is connected, a powder B hopper 32 which stores a dialysis powder B, and a dialysis powder B feeder 33 which feeds a dialysis powder B into this solution B tank 31.
The above-described solution B passage 8 is such that the discharge passage 8a is provided with a third liquid feeding pump P3 which feeds the liquid of the solution B tank 31, a solution B circulation passage 34 which branches off from the discharge passage 8a in a position on the downstream side of the third liquid feeding pump P3 and is connected to the solution B tank 31, and a second relief valve RV2 provided in the solution B circulation passage 34, and that the inlet passage 8b is provided with a fifth on-off valve SV5.
Because the configuration of the powder B dissolving means 2 and the operation thereof are the same as in the above-described powder A dissolving means 1, a detailed description of the powder B dissolving means 2 is omitted. It is possible to use a conventional, publicly-known dialysis powder dissolving device as such powder dissolving means.

The above-described weighing means 4 is an airtight vessel whose interior is partitioned by a diaphragm 4a and has a solution A chamber 4A into which the solution A flows via the solution A passage 7 and a solution B chamber 4B into which the solution B flows via the solution B passage 8, and the solution A chamber 4A and solution B chamber 4B have the same capacity.
The above-described solution A/solution B passage 9 joins after being connected to the above-described solution A chamber 4A and solution B chamber 4B, and then the solution A/solution B passage 9 branches off again and is connected to both ends of the above-described reciprocating pump 5. A fourth liquid feeding pump P4 and a flow detection sensor FS are provided in the above-described part where the branched sections of the solution A/solution B passage 9 join.
A sixth on-off valve SV6 is provided between the fourth liquid feeding pump P4 and the solution A chamber 4A, and a seventh on-off valve SV7 is provided between the fourth liquid feeding pump P4 and the solution B chamber 4B. Between the fourth liquid feeding pump P4 and the reciprocating pump 5, an eighth on-off valve SV8 is provided in one branched passage and the ninth on-off valve SV9 is provided in the other branched passage.
Because of this configuration, for example, when the second liquid feeding pump P2 feeds the solution A, with the fourth and seventh on-off valves SV4, SV7 kept open and the fifth and sixth on-off valves SV5, SV6 kept closed, the solution A is supplied to the solution A chamber 4A of the weighing means 4 and the diaphragm 4a becomes deformed, with the result that the volume of the solution A chamber 4A increases.
On that occasion, the solution B is discharged from the solution B chamber 4B due to the action of the fourth liquid feeding pump P4, and this solution B is fed to the reciprocating pump 5 via either of the branched passages of the eighth and ninth on-off valves SV8, SV9, which are open.
And the solution A is supplied to the solution A chamber 4A until the solution B chamber 4B disappears and conversely, the solution B is supplied to the solution B chamber 4B until the solution A chamber 4A disappears. By performing these operations alternately, it is possible to feed the solution A and the solution B in the same amount alternately from the weighing means 4 to the reciprocating pump 5.
In this embodiment as described above, the liquid feeding means of the present invention is composed of the sixth, seventh, eighth and ninth on-off valves SV6, SV7, SV8, SV9 which open and close the passages between the weighing means 4 and the reciprocating pump 5 as well as the fourth liquid feeding pump P4.
When the solution A and the solution B are discharged from the solution A chamber 4A or the solution B chamber 4B due to the inflow pressure by the above-described second and third liquid feeding pumps P2, P3, it is also possible to omit the above-described fourth liquid feeding pump P4.
In this case, the second and third pumps P2, P3 may also be provided in the inlet passages 8b, 9b, respectively, on the dialysate preparation device 3 side.

The above-described reciprocating pump 5 is provided with a cylindrical tank 41, a piston 42 which reciprocates while sliding within this tank 41, a rod 43 provided so as to penetrate this piston 42, and limit switches 44 provided in positions spaced from the above-described tank 41.
In the interior of the above-described tank 41, mixing chambers 41a, 41b are formed on the left and right sides of the above-described piston 42, and the solution A/solution B passage 9 which branches off and the above-described dialysate passage 10 which branches off similarly are connected to the left and right mixing chambers 41a, 41b .
The above-described dialysate passage 10 joins in a position downstream of the mixing chambers 41a, 41b, and there are provided two concentration sensors CD which measure the concentration of the dialysate, a discharge liquid passage 45 which branches off downstream of these concentration sensors CD, a first motor valve MV1 provided downstream of the branch position of the discharge liquid passage 45, and a second motor valve MV2.
The dialysate passage 10 on the downstream side of the first motor valve MV1 is connected to a plurality of dialysis devices, which are not shown, after branching off, and on the downstream side of the second motor valve MV2 there is a discharge liquid port, which is connected to a discharge liquid pipe, which is not shown.
The above-described eighth and ninth on-off valves SV8, SV9 provided in the above-described solution A/solution B passage 9 are such that only either of the two is adapted to be opened, and for this reason, it is ensured that the above-described reciprocating pump 5 is such that the solution A and the solution B flow into only either of the mixing chambers 41a, 41b.
Because the solution A and the solution B alternately flow in the same number of times, for example, in the mixing chamber 41b, one of the two mixing chambers, the solution A and the solution B are mixed and a dialysate is prepared and furthermore the piston 42 is pressed to the other mixing chamber 41a side, which is the opposite side, with the result that it is ensured that from the other mixing chamber 41a, a dialysate which is prepared beforehand is discharged to the dialysate passage 10.
The above-described rod 43 projects to both sides of the tank 41, as shown in the drawing, and it is ensured that when the piston 42 reciprocates and moves to a prescribed right-hand end or the left-hand end, the piston 42 comes into contact with either of the limit switches 44 and outputs a required alarm.

Hereinafter, a description will be given of the dialysate preparation method using the dialysate preparation device 3.
First, a dialysate prepared in this embodiment will be described. In this embodiment, a dialysate is prepared by using LYMPACK TA3 (registered trademark) made by Nipro Co., Ltd. Agent A (a dialysis powder A) of this drug product contains sodium chloride and potassium chloride and Agent B (a dialysis powder B) contains sodium hydrogencarbonate.
In the powder A dissolving means 1 of this embodiment, the solution A is prepared with a concentration of approximately 1.703 w/v%, and in the powder B dissolving means 2, the solution B is prepared with a concentration of approximately 0.419 w/v%.
By preparing the solution A and the solution B with these concentrations, it is possible to obtain a dialysate having the same compositions as a dialysate prepared by the original method by mixing the solution A and the solution B at proportions of 1:1 even without adding purified water to them.
In the above-described original method, it is necessary that 9 L of an undiluted liquid A be prepared by dissolving 2682 g of Agent A in purified water beforehand and that 11.34 L of an undiluted liquid B be prepared by dissolving 661.6 g of Agent B in purified water. In this case, the concentration of Agent A in the above-described undiluted liquid A is 29.8 w/v%, and the concentration of Agent B in the above-described undiluted liquid B is approximately 5.83 w/v%.
And by using the undiluted liquid A and the undiluted liquid B prepared in this manner, the above-described undiluted liquid A, undiluted liquid B and purified water are mixed at the ratio of 1:1.26:32.74, whereby a dialysate is prepared.

Next, a description will be given on the assumption that in Figure 1, the dialysate preparation device 3 is already in a usual operating condition.
In Figure 1, when the second on-off valve SV2 of the purified water passage 6 is closed and at the same time the third on-off valve SV3 is opened, purified water is supplied to the powder B dissolving means 2 without the supply of purified water to the powder A dissolving means 1.
In the powder A dissolving means 1, the preparation of the solution A is stopped and the above-described fourth on-off valve SV4 is opened, as a result of which, the solution A fed by the second liquid feeding pump P2 flows into the solution A chamber 4A of the above-described weighing means 4 without circulating in the solution A circulation passage 24.
On the other hand, in the powder B dissolving means 2 the preparation of the solution B is performed and the above-described fifth on-off valve SV5 is closed, as a result of which, it is ensured that the solution B fed by the third liquid feeding pump P3 circulates in the above-described solution B circulation passage 34 and solution B tank 31, thereby stirring the dialysis powder B and purified water.

Also in Figure 1, the sixth on-off valve SV6 of the solution A/solution B passage 9 of the dialysate preparation device 3 is closed and the seventh on-off valve SV7 is opened, as a result of which, it is ensured that the solution A which has flowed through the above-described solution A passage 7 flows into the solution A chamber 4A of the weighing means 4, and that this solution A is not discharged to the solution A/solution B passage 9.
In the weighing means 4, the seventh on-off valve SV7 is opened, as a result of which, the solution B is fed from the solution B chamber 4B to the reciprocating pump 5, and the solution A flows into the solution A chamber 4A, as a result of which, the diaphragm 4a is pressed, leading to an increase in the volume of the solution A chamber 4A.
Because the solution B chamber 4B of the weighing means 4 disappears and the solution A chamber 4A obtains a maximum volume, a prescribed amount of the solution A is weighed by the weighing means 4 and the solution B is fed to the reciprocating pump 5 in an amount equal to this amount.

Furthermore in Figure 1, the eighth on-off valve SV8 of the solution A/solution B passage 9 is closed and the ninth on-off valve SV9 is opened, as a result of which, the solution B discharged from the solution B chamber 4B of the above-described weighing means 4 is fed by the above-described fourth liquid feeding pump P4 and flows into the right-hand mixing chamber 41b of the above-described reciprocating pump 5 as seen in the drawing via the ninth on-off valve SV9.
Because the solution B flows in, the piston 42 moves leftward as seen in the drawing, as a result of which, a dialysate stored in the left-hand mixing chamber 41a as seen in the drawing is discharged to the dialysate passage 10.
The concentration of the discharged dialysate is measured by the concentration sensors CD provided in the dialysate passage 10. If the concentration of the dialysate is appropriate, the above-described first motor valve MV1 is opened and the second motor valve MV2 of the discharge liquid passage 45 is closed, as a result of which, the dialysate is fed to dialysis devices via the dialysate passage 10.
Conversely when there is an abnormality in the concentration of the dialysate, the above-described first motor valve MV1 is closed and the second motor valve MV2 of the discharge liquid passage 45 is opened, as a result of which, the dialysate is discharged via the discharge liquid passage 45.
It is ensured that at the start of the operation of the dialysate preparation device 3, the liquid is discharged to the discharge liquid pipe by opening the second motor valve MV2 until the dialysate with a prescribed concentration becomes stably supplied.

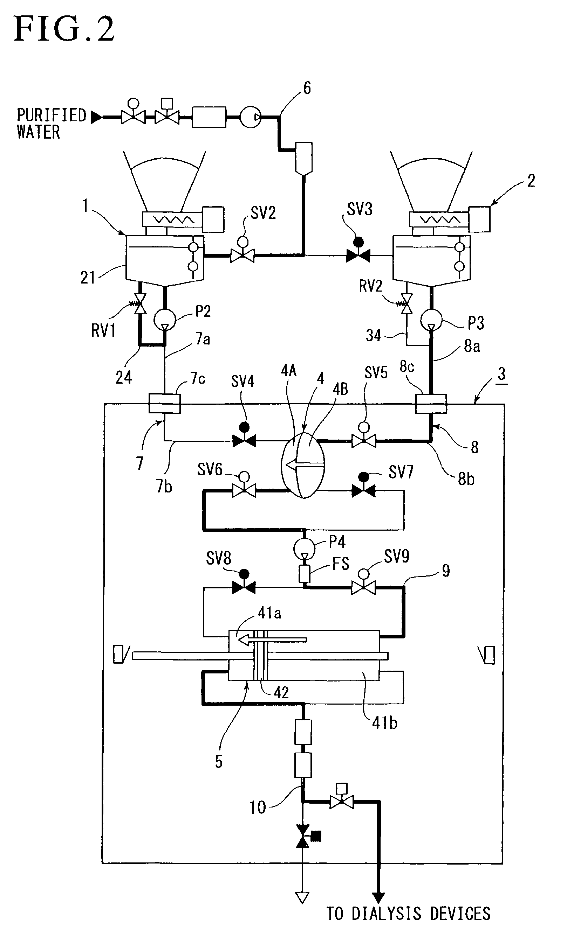

Figure 2 shows a condition occurring after a lapse of a prescribed time from the condition of Figure 1 above. Concretely, when in the weighing means 4 in Figure 1 above the solution B chamber 4B disappears by being pushed by the diaphragm 4a and the flowing of the solution B is not detected any more by the flow detection sensor FS of the above-described solution A/solution B passage 9, it is ensured that each of the on-off valves SV4 to 7 is changed from the condition of Figure 1 to the condition of Figure 2.
In Figure 2, the second on-off valve SV2 of the purified water passage 6 is opened and at the same time the third on-off valve SV3 is closed, as a result of which, purified water is supplied to the powder A dissolving means 1 and the supply of purified water to the powder B dissolving means 2 is stopped.
And in the powder A dissolving means 1, the preparation of the solution A is started and the above-described fourth on-off valve SV4 is closed. Therefore, it is ensured that the solution A fed by the second liquid feeding pump P2 circulates in the solution A circulation passage 24 and stirs the dialysis powder A and purified water in the solution A tank 21.
On the other hand, because in the powder B dissolving means 2, the preparation of the solution B is stopped and the above-described fifth on-off valve SV5 is opened, the solution B fed by the third liquid feeding pump P3 flows into the solution B chamber 4B of the above-described weighing means 4 without circulating in the above-described solution B circulation passage 34.

Also in Figure 2, the sixth on-off valve SV6 of the solution A/solution B passage 9 is opened and the seventh on-off valve SV7 is closed, as a result of which, it is ensured that the solution B which has flowed through the above-described solution B passage 8 flows into the solution B chamber 4B of the weighing means 4, and that this solution B is prevented from entering the solution A/solution B passage 9.
In the weighing means 4, the solution A is fed from the solution A chamber 4A to the reciprocating pump 5 by opening the sixth on-off valve SV6, and the diaphragm 4a is pushed by the inflow of the solution B into the solution B chamber 4B, resulting in an increase in the volume of the solution B chamber 4B.
And because the solution A chamber 4A of the weighing means 4 disappears and the solution B chamber 4B obtains a maximum volume, a prescribed amount of the solution B is weighed by the weighing means 4 and the solution B is discharged in an amount equal to this amount.

Furthermore in Figure 2, as with the case of Figure 1, the eighth on-off valve SV8 of the solution A/solution B passage 9 is closed and the ninth on-off valve SV9 is opened, as a result of which, the solution A discharged from the above-described solution A chamber 4A is caused, by the above-described fourth liquid feeding pump P4, to flow into the right-hand mixing chamber 41b of the above-described reciprocating pump 5 as seen in the drawing via the ninth on-off valve SV9. In the mixing chamber 41b, because in Figure 1 the solution B has already flowed in and the same amount of the solution A as the amount of the solution B flows into this mixing chamber 41b, within the mixing chamber 41b the solution A and the solution B are mixed in proportions of 1:1.
At this time, because the powder A dissolving means 1 and the powder B dissolving means 2 have already prepared the solution A and the solution B with the above-described concentrations, a dialysate can be obtained simply by mixing the solution A and the solution B even if purified water is not supplied anew to this mixing chamber 41b.
Furthermore, because the solution A flows into the mixing chamber 41b, the piston 42 moves further leftward as seen in the drawing, whereby the dialysate in the left-hand mixing chamber 41a as seen in the drawing is discharged to the dialysate passage 10, and is supplied to dialysis devices as described above.

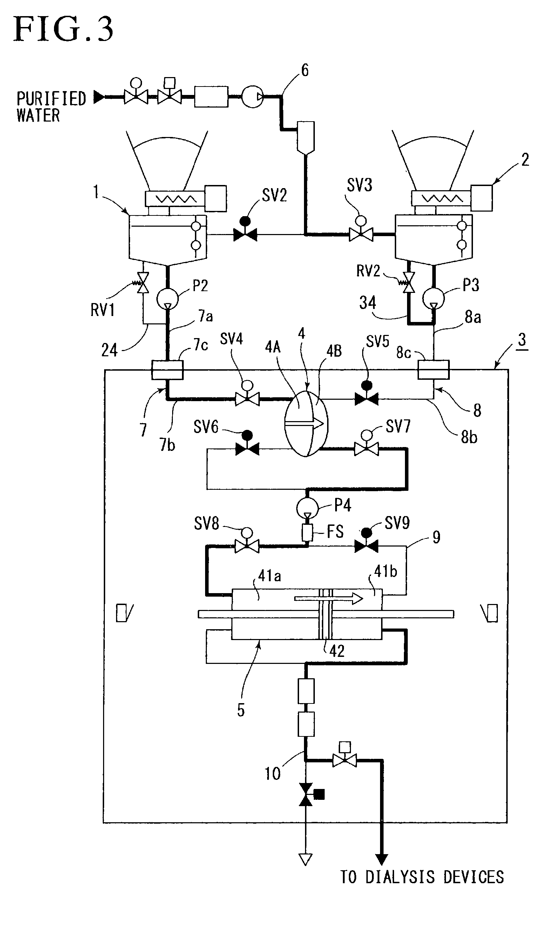

Figure 3 shows a condition occurring after a lapse of a prescribed time from the condition of Figure 2 above.
Concretely, when in the weighing means 4 in Figure 2 above the solution A chamber 4A disappears by being pushed by the diaphragm 4a and the flowing of the solution A is not detected any more by the flow detection sensor FS of the above-described solution A/solution B passage 9, it is ensured that each of the on-off valves SV4 to 7 is changed from the condition of Figure 2 to the condition of Figure 3.
In this condition of Figure 3, like in the condition of Figure 1 above, the preparation of the solution A is stopped in the powder A dissolving means 1, whereas in the powder B dissolving means 2, the preparation of the solution B is being performed and it is ensured that the solution A is supplied to the weighing means 4.
In the weighing means 4, the solution A is supplied to the solution A chamber 4A and the solution B is discharged from the solution B chamber 4B. Also at this time, until the solution B chamber 4B disappears, a given amount of the solution A is weighed by the weighing means 4 and on the other hand, it is ensured that the solution B is fed in an amount equal to the amount of the solution A.

Unlike the conditions of Figures 1 and 2, in the condition of Figure 3, the eighth on-off valve SV8 of the solution A/solution B passage 9 is opened and the ninth on-off valve SV9 is closed.
That is, in Figures 1 and 2, the solution A and the solution B are weighed in the same number of times (once each in this embodiment), and the solution A and the solution B flow in the same amount once each into the right-hand mixing chamber 41b as seen in the drawings and a prescribed amount of a dialysate was prepared in this mixing chamber 41b. Therefore, in Figure 3, it is ensured that the solution B is supplied to the left-hand mixing chamber 41a.
As a result of this, the solution B fed from the solution B chamber 4B of the above-described weighing means 4 flows into the left-hand mixing chamber 41a of reciprocating pump 5 as shown in the drawing by means of the above-described fourth liquid feeding pump P4 via the eighth on-off valve SV8.
Because the solution B flows in, the piston 42 moves rightward as seen in the drawing, with the result that the dialysate in the right-hand mixing chamber 41b as seen in the drawing, which was prepared in Figure 2, is fed to dialysis devices via the dialysate passage 10.

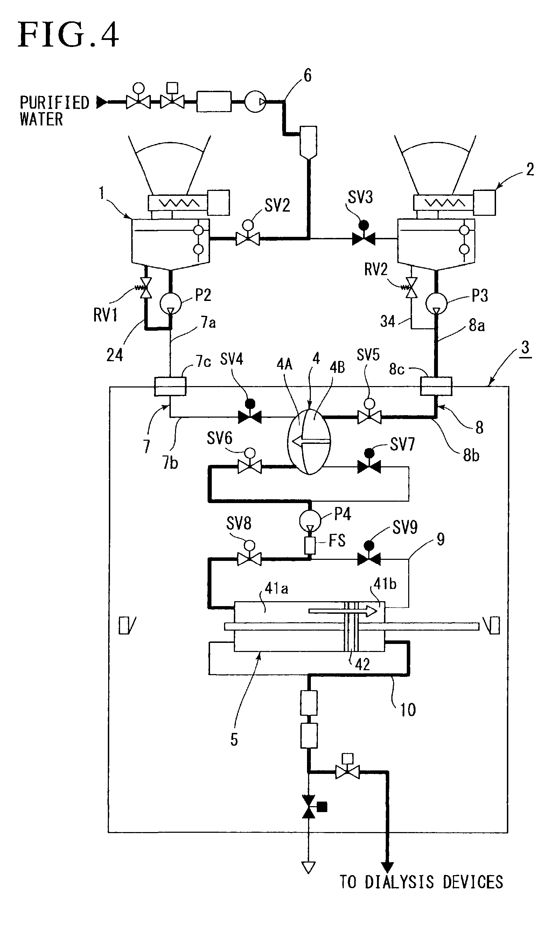

Figure 4 shows a condition occurring after a lapse of a prescribed time from the condition of Figure 3 above.
Concretely, when in the weighing means 4 in Figure 3 above the solution B chamber 4B disappears by being pushed by the diaphragm 4a and the flowing of the solution B is not detected any more by the flow detection sensor FS of the above-described solution A/solution B passage 9, it is ensured that a changeover is made from the condition of Figure 3 to the condition of Figure 4.
In this condition of Figure 4, like in the condition of Figure 2 above, the powder A dissolving means 1 performs the preparation of the solution A, whereas the powder B dissolving means 2 stops the preparation of the solution B and it is ensured that the solution B is supplied to the weighing means 4.
In the weighing means 4, the solution B is supplied to the solution B chamber 4B and the solution A is discharged from the solution A chamber 4A. Also at this time, until the solution A chamber 4A disappears, a given amount of the solution B capable of being housed in the weighing means 4 composed of an airtight vessel is measured and it is ensured that the solution A is fed in an amount equal to the amount of the weighed solution B.

Next, in the condition of Figure 4, as with the condition of Figure 3, the eighth on-off valve SV8 of the solution A/solution B passage 9 is opened and the ninth on-off valve SV9 is closed, as a result of which, the solution A discharged from the solution A chamber 4A flows, by means of the above-described liquid feeding pump P3, into the left-hand mixing chamber 41a of the above-described reciprocating pump 5 as seen in the drawing via the eighth on-off valve SV8.
In this mixing chamber 41a, because the solution B has already flowed in Figure 3 and the same amount of the solution A as the solution B flows into this mixing chamber 41a, within this mixing chamber 41a the solution A and the solution B are mixed in proportions of 1:1 and a dialysate is prepared.
Furthermore, because the solution A flows in, the piston 42 moves rightward as seen in the drawing, with the result that the dialysate in the right-hand mixing chamber 41b as seen in the drawing is fed to dialysis devices via the dialysate passage 10.
When the solution A and the solution B have flowed into the left-hand mixing chamber 41a as shown in the drawing in the same number of times, a changeover to the condition of Figure 1 occurs again, and it is ensured that a dialysate can be continuously supplied to dialysis devices while a changeover is being made subsequently from the condition of Figure 1 to the conditions of Figures 2 to 4.

According to the embodiment described above, the above-described powder A dissolving means 1 and powder B dissolving means 2 prepare beforehand the solution A and the solution B with the above-described concentrations, and hence if in the dialysate preparation device 3, the weighing means 4 weighs the same amount of the solution A and the solution B for the same number of times and feeds the solution A and the solution B to the reciprocating pump 5, the solution A and the solution B are mixed in proportions of 1:1 and a dialysate can be obtained without the addition of purified water. Thus, it is possible to prepare a dialysate easily.
Also, because weighing is performed by an airtight vessel which is partitioned into two by the diaphragm 4a as the above-described weighing means 4, it is possible to alternately feed the solution A and the solution B to the reciprocating pump 5 in the same amount in proportions of 1:1 and it is possible to prepare a dialysate without precisely controlling amounts of solutions which are fed by use of a precision pump and the like.
In contrast to this, according to conventional dialysate preparation devices 3, it is necessary to mix the undiluted liquid A, the undiluted liquid B and purified water at the prescribed ratio (1:1.26:32.74), and it is necessary that these be precisely weighed, posing the problems that the work related to the preparation of a dialysate becomes complicated and that as a result of this, the configuration becomes complex.
Also, because a dialysate is obtained by mixing the above-described solution A and solution B in the above-described reciprocating pump 5, the configuration becomes simple compared to conventional art and it is possible to miniaturize equipment.
Furthermore, because the weighing means 4 and the reciprocating pump 5 are provided with the variable-volume mixing chambers 41a, 41b within an airtight vessel, the inside liquid does not come into contact with the outside air and hence the bacteria and microparticles in the air do not mix into the prepared dialysate.
The above-described solution A and solution B can be prepared by using conventional, publicly-known powder A dissolving devices and powder B dissolving devices and the present invention can be introduced at low cost by using equipment which is already installed in hospitals and the like.

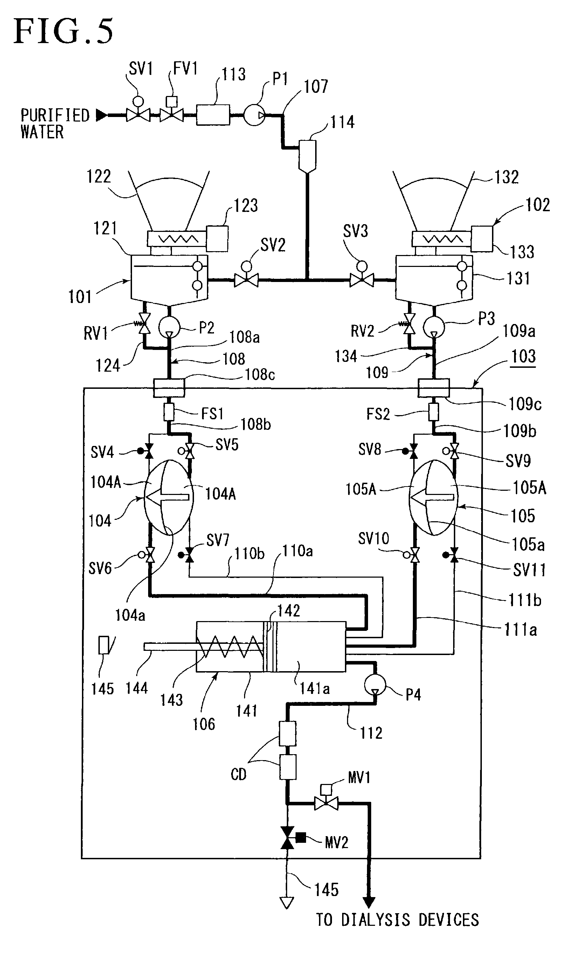

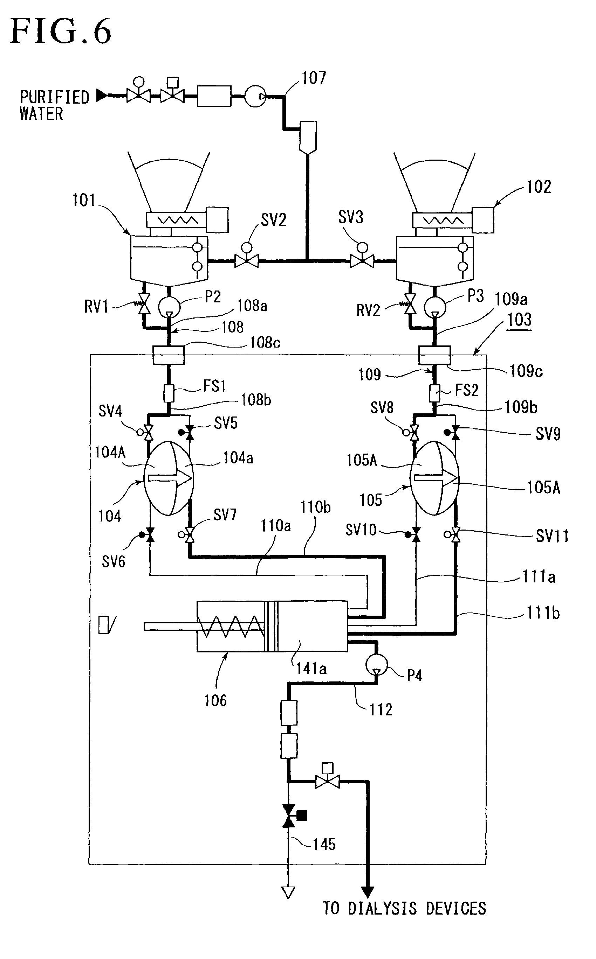

Figures 5 and 6 show the second embodiment of the present invention and show a powder A dissolving means 101 which prepares a solution A by dissolving a dialysis powder A in purified water, a powder B dissolving means 102 which prepares a solution B by dissolving a dialysis powder B in purified water, and a dialysate preparation device 103 which prepares a dialysate by mixing the solution A and the solution B.
Because in the description which will be given below, the above-described powder A dissolving means 101 and powder B dissolving means 102 have the same configuration as in the first embodiment, detailed descriptions thereof are omitted and in the drawings, symbols obtained by adding 100 to the symbols used in the first embodiment are used.
The above-described dialysate preparation device 103 is provided with a solution A airtight vessel 104 and a solution B airtight vessel 105 as weighing means which weighs the solution A and solution B supplied from the powder A dissolving means 101 and the powder B dissolving means 102, liquid feeding means which feeds the solution A and solution B weighed by them, and an accumulator tank 106 which prepares a dialysate by mixing the solution A and the solution B and supplies this dialysate to dialysis devices, which are not shown.
The above-described solution A airtight vessel 104 and solution B airtight vessel 105 have the same solution housing capacity and both can weigh the same amount.
A purified water passage 107 which supplies purified water is connected to the above-described powder A dissolving means 101 and powder B dissolving means 102. A solution A supply passage 108 is arranged between the powder A dissolving means 101 and the solution A airtight vessel 104. A solution B supply passage 109 is arranged between the powder B dissolving means 102 and the solution B airtight vessel 105. Solution A discharge passages 110a, 110b are arranged between the solution A airtight vessel 104 and the accumulator tank 106. Solution B discharge passages 111a, 111b are arranged between the solution B airtight vessel 105 and the accumulator tank 106. A dialysate passage 112 is arranged between the accumulator tank 106 and the dialysis device. The fourth liquid feeding pump P4 which feeds a dialysate from the accumulator tank 106 to the downstream side is arranged in this dialysate passage 112.
And the above-described purified water passage 107 is provided with, in order from the upstream side, a first on-off valve SV1, a flow regulating valve FV1, a heater 113, a first liquid feeding pump P1 and a deaeration tank 114. A second on-off valve SV2 is provided on the powder A dissolving means 101 side of the branched purified water passage 107, and a third on-off valve SV3 is provided on the powder B dissolving means 102 side.

The solution A airtight vessel 104 is partitioned by a diaphragm 104a into two chambers, which are solution A chambers 104A having the same capacity.
The above-described solution A supply passage 108 is composed of a discharge passage 108a on the powder A dissolving means 101 side, an inlet passage 108b on the dialysate preparation device 103 side, and a connection 108c which connects them.
A second liquid feeding pump P2 is provided in the above-described discharge passage 108a, a solution A circulation passage 124 branches off downstream of the second liquid feeding pump P2 and is connected to a solution A tank 121, and a relief valve RV1 is provided in this solution A circulation passage 124.
The inlet passage 108b is provided with a solution A flow detecting sensor FS1 which detects whether or not the solution A flows, and the inlet passage 108b branches off into two on the downstream side of this solution A flow detecting sensor FS1.
Sections of the branched inlet passage 108b are each connected to each of the solution A chambers 104A of the above-described solution A airtight vessel 104, and a fourth on-off valve and a fifth on-off valve SV4, SV5 are provided so as to correspond to each solution A chamber 104A.
Furthermore, the above-described solution A discharge passages 110a, 110b are connected to each solution A chamber 104A, a sixth on-off valve SV6 is provided in the solution A discharge passages 110a, and a seventh on-off valve SV7 is provided in the solution A discharge passages 110b.
The flow rate of the liquid capable of being fed in the above-described inlet passage 108b is set at a flow rate smaller than the liquid flow rate of the above-described second liquid feeding pump P2, and for this reason, it is ensured that in the discharge passage 108a, part of the solution A circulates to a solution A tank 121 via the above-described solution A circulation passage 124, with the result that the stirring of the dialysis powder A and purified water is performed.

As with the above-described solution A airtight vessel 104, the solution B airtight vessel 105 is partitioned by a diaphragm 105a into two chambers, which are solution B chambers 105A having the same capacity. The capacity of these solution B chambers 105A is also equal to the capacity of the solution A chamber 104A of the solution A airtight vessel 104.
The above-described solution B supply passage 109 is composed of a discharge passage 109a on the powder B dissolving means 102 side, an inlet passage 109b on the dialysate preparation device 103 side, and a connection 109c which connects them.
A third liquid feeding pump P3 is provided in the above-described discharge passage 109a, a solution B circulation passage 134 branches off downstream of the third liquid feeding pump P3 and is connected to a solution B tank 131, and a relief valve RV2 is provided in this solution B circulation passage 134.
The inlet passage 109b is provided with a solution B flow detecting sensor FS2 which detects whether or not the solution B flows, and the inlet passage 109b branches off into two on the downstream side of this solution B flow detecting sensor FS2.
Sections of the branched inlet passage 109b are each connected to each solution B chamber 105A of the above-described solution B airtight vessel 105, and an eighth on-off valve and a ninth on-off valve SV8, SV9 are provided so as to correspond to each solution B chamber 105A of the above-described solution B airtight vessel 105.
Furthermore, the above-described solution B discharge passages 111a, 111b are connected to each solution B chamber 105A, a tenth on-off valve SV10 is provided in the solution B discharge passages 111a, and an eleventh on-off valve SV11 is provided in the solution B discharge passage 111b.
The flow rate of the liquid capable of being fed in the above-described inlet passage 109b is set at a flow rate smaller than the liquid flow rate of the above-described third liquid feeding pump P3, and for this reason, it is ensured that in the discharge passage 109a, part of the solution B circulates to a solution B tank 131 via the above-described solution B circulation passage 134, with the result that the stirring of the dialysis powder B and purified water is performed.

The above-described accumulator tank 106 is provided with a cylindrical tank 141, a piston 142 which reciprocates within this tank 141 while sliding, a spring 143 as urging means which urges this piston 142, a rod 144 provided in the above-described piston 142, and a limit switch 145 provided in a position spaced from the above-described tank 141.
The above-described tank 141 is such that a mixing chamber 141a is formed by the above-described piston 142 on the right side, as seen in the drawing, which is opposite to the side where the spring 143 and the rod 144 are provided, and to this mixing chamber 141a there are each connected the above-described solution A discharge passages 110a, 110b and solution B discharge passages 111a, 111b as well as the dialysate passage 112.
The above-described spring 143 urges the piston 142 in the direction in which the volume of the mixing chamber 141a is reduced constantly (the right side as seen in the drawing), and when a dialysate in the mixing chamber 141a is discharged by the fourth liquid feeding pump P4 to the dialysate passage 112, the piston 142 moves rightward as seen in the drawing by the urge of the spring 143 so as to reduce the volume of the mixing chamber 141a, with the result that it is ensured that the interior of the mixing chamber 141a is constantly kept filled with the dialysate and out of contact with the atmosphere.
The above-described rod 144 protrudes to the left side of the tank 141 as seen in the drawing, and is provided in such a manner that the piston 142 comes into contact with the limit switch 145 when the piston 142 moves to the leftward end as seen in the drawing, whereby control means is adapted to output a prescribed alarm when the limit switch 145 works.

In this configuration, as shown in Figure 5, the fifth and sixth on-off valves SV5, SV6 are opened at the same time the fourth and seventh on-off valves SV4, SV7 are closed and the solution A is fed to the accumulator tank 106. At the same time, the ninth and tenth on-off valves SV9, SV10 are opened at the same time the eighth and eleventh on-off valves SV8, SV11 are closed, whereby the solution B is fed to the accumulator tank 106.
In this state, the solution A is supplied to the right-hand solution A chamber 104A of the solution A airtight vessel 104 as seen in the drawing and is discharged from the left-hand solution A chamber 104A as seen in the drawing and is fed to the accumulator tank 106 via the sixth on-off valve SV6 of the solution A discharge passage 110a.
At the same time, the solution B is supplied to the right-hand solution B chamber 105A of the solution B airtight vessel 105 as seen in the drawing and is discharged from the left-hand solution B chamber 105A as seen in the drawing and is fed to the accumulator tank 106 via the tenth on-off valve SV10 of the solution B discharge passage 111a.
As described above, in this embodiment, it is ensured that liquid feeding to the accumulator tank 106 is performed simultaneously from both of the solution A airtight vessel 104 and the solution B airtight vessel 105. It is ensured that the left-hand solution A chamber 104A and solution B chamber 105A of the solution A airtight vessel 104 and solution B airtight vessel 105, as seen in the drawing, disappear simultaneously, and that when a changeover is made in the open-closed condition of each of the above-described on-off valves, this time the solution A and the solution B are supplied to the left-side solution A chamber 104A and solution B chamber 105A, respectively, as seen in the drawing.
And this is performed alternately, the solution A and the solution B are weighed in the same amount for the same number of times by means of the solution A airtight vessel 104 and the solution B airtight vessel 105 and are fed to the accumulator tank 106, whereby it is possible to mix the solution A and the solution B in the same amount in proportions of 1:1.
In this embodiment as described above, the liquid feeding means of the present invention is composed of the on-off valves which open and close the flow passages ranging from the solution A airtight vessel 104 and the solution B airtight vessel 105 to the accumulator tank 106, SV6, SV7, SV10, SV11, and the fourth liquid feeding pump P4.

Hereinafter, a description will be given of the dialysate preparation method using this dialysate preparation device 103. Also here, the description will be given on the assumption that in Figure 5, the powder A dissolving means 101, the powder B dissolving means 102 and the dialysate preparation device 103 are already in a usual operating condition.
First, as in the first embodiment described above, in the powder A dissolving means 101 and the powder B dissolving means 102, the solution A is prepared with a concentration of approximately 1.703 w/v% and the solution B is prepared with a concentration of approximately 0.419 w/v%.
In this embodiment, it is ensured that the solution A and the solution B are basically prepared continuously during the operation of the dialysate preparation device 103, and it is ensured that the above-described second and third liquid feeding pumps P2, P3 constantly feed the solution A and the solution B at prescribed flow rates.
However, because parts of the solution A and the solution B circulate via the solution A circulation passage 124 and the solution B circulation passage 134, respectively, it is ensured that in the solution A tank 121 and the solution B tank 131, the dialysis powder A and the dialysis powder B, and purified water are stirred, respectively.

In Figure 5, the fourth on-off valve SV4 positioned upstream of the solution A airtight vessel 104 is closed and at the same time the fifth on-off valve SV5 is opened; the sixth on-off valve SV6 positioned downstream is opened and at the same time the seventh on-off valve SV7 is closed.
For this reason, the solution A passing through the solution A flow detecting sensor FS1 flows into the right-hand solution A chamber 104A as seen in the drawing via the fifth on-off valve SV5, and conversely, from the left-hand solution A chamber 104A as seen in the drawing, the solution A is fed to the above-described accumulator tank 106 via the sixth on-off valve SV6.
On the other hand, the eighth on-off valve SV8 positioned upstream of the solution B airtight vessel 105 is closed and at the same time the ninth on-off valve SV9 is opened; the tenth on-off valve SV10 positioned downstream is opened and at the same time the eleventh on-off valve SV11 is closed.
For this reason, the solution B passing through the solution B flow detecting sensor FS2 flows into the right-hand solution B chamber 105A as seen in the drawing via the ninth on-off valve SV9, and conversely, from the left-hand solution B chamber 105A as seen in the drawing, the solution B is fed to the above-described accumulator tank 106 via the tenth on-off valve SV10.
Because the left-hand solution A chamber 104A of the solution A airtight vessel 104 as seen in the drawing disappears and the right-hand solution A chamber 104A as seen in the drawing obtains a maximum volume, a prescribed amount of the solution A is weighed in the solution A airtight vessel 104 and in the same manner, a prescribed amount of the solution B is measured also in the solution B airtight vessel 105.
Furthermore, as the solution A chamber 104A of the solution A airtight vessel 104 and the solution B chamber 105A of the solution B airtight vessel 105 have the same capacity, the amount of the solution A weighed by the solution A airtight vessel 104 and the amount of the solution B weighed by the solution B airtight vessel 105 become equal to each other, and it is ensured that the solution A and the solution B are mixed in proportions of 1:1 by simultaneously feeding the solutions.

The solution A and the solution B flow simultaneously in the same amount into the accumulator tank 106 from the above-described solution A airtight vessel 104 and the solution B airtight vessel 105, and the solution A and the solution B are prepared with the above-described concentrations so that a dialysate can be obtained without the addition of purified water. Therefore, a dialysate is prepared immediately in the mixing chamber 141a.
When the solution A and the solution B flow in, the piston 142 of the accumulator tank 106 moves leftward as seen in the drawing against the urging force of the spring 143, whereas because the dialysate is discharged by the fourth liquid feeding pump P4, the piston 142 moves rightward as seen in the drawing under the urging force of the spring 143.
And it is ensured that the dialysate discharged from the accumulator tank 106 is supplied to the above-described dialysis device or discharged via the above-described discharge liquid passage 145 after the measurement of the concentration with the concentration sensor CD provided in the dialysate passage 112.

Figure 6 shows a condition occurring after a lapse of a prescribed time from the condition of Figure 5 above.
Concretely, it is ensured that a changeover is made from the condition of Figure 5 to the condition of Figure 6 when the left-hand solution A chamber 104A and solution B chamber 105 as seen in the drawing in the above-described solution A airtight vessel 104 and solution B airtight vessel 105 disappear from the condition shown in Figure 5 by being pushed by the diaphragms 104a, 105a and the solution A flow detecting sensor FS1 and the solution B flow detecting sensor FS2 do not detect the flowing of the solution A and the solution B any more.
In this condition, the fourth on-off valve SV4 positioned upstream of the solution A airtight vessel 104 is opened and at the same time the fifth on-off valve SV5 is closed; the sixth on-off valve SV6 positioned downstream is closed and at the same time the seventh on-off valve SV7 is opened.
For this reason, the solution A passing through the solution A flow detecting sensor FS1 flows into the left-hand solution A chamber 104A as seen in the drawing via the fourth on-off valve SV4, and conversely, from the right-hand solution A chamber 104A as seen in the drawing, the solution A is fed to the above-described accumulator tank 106 via the seventh on-off valve SV7.
On the other hand, the eighth on-off valve SV8 positioned upstream of the solution B airtight vessel 105 is opened and at the same time the ninth on-off valve SV9 is closed; the tenth on-off valve SV10 positioned downstream is closed and at the same time the eleventh on-off valve SV11 is opened.
For this reason, the solution B passing through the solution B flow detecting sensor FS2 flows into the left-hand solution B chamber 105A as seen in the drawing via the eighth on-off valve SV8, and conversely, from the right-hand solution B chamber 105A as seen in the drawing, the solution B is fed to the above-described accumulator tank 106 via the eleventh on-off valve SV11.
It is ensured that at this time, the above-described fourth to eleventh on-off valves SV4 to SV11 undergo a changeover simultaneously; the amount of the solution A fed from the solution A airtight vessel 104 and the solution B fed from the solution B airtight vessel 105 are equal to each other, in the accumulator tank 106 the solution A and the solution B are supplied in the same amount, as a result of which, in the mixing chamber 141a the solution A and the solution B are mixed in proportions of 1:1, a dialysate is prepared immediately, and this dialysate is supplied by the above-described fourth liquid feeding pump P4 to dialysis devices.

According to the above-described embodiment, because the solution A and the solution B are prepared beforehand with the above-described concentrations and the solution A and the solution B are supplied simultaneously to the accumulator tank 106 in proportions of 1:1, a dialysate can be obtained without the addition of purified water and it is possible to prepare a dialysate easily.
Also, because it is only necessary for the above-described solution A and solution B to be mixed in the accumulator tank 106, the configuration becomes simple compared to the conventional art in which conventional mixing tanks and a storage tank have to be installed, and it becomes possible to miniaturize equipment.
Furthermore, the above-described solution A and solution B can be prepared by using conventional, publicly-known powder A dissolving means and powder B dissolving means and the present invention can be introduced at low cost by using apparatus which is already installed in hospitals and the like.

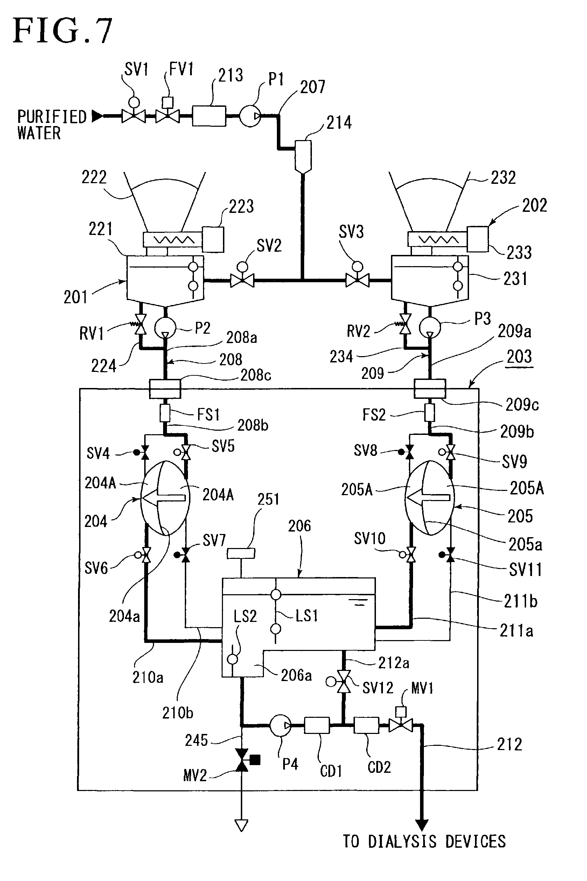

Figure 7 shows the third embodiment of the present invention and shows a powder A dissolving means 201 which prepares a solution A by dissolving a dialysis powder A in purified water, a powder B dissolving means 202 which prepares a solution B by dissolving a dialysis powder B in purified water, and a dialysate preparation device 203 which prepares a dialysate by mixing the solution A and the solution B.
In the description which will be given below, detailed descriptions of parts having the same configuration as in the second embodiment are omitted and in the drawing, symbols obtained by adding 100 to the symbols used in the second embodiment are used.
The above-described dialysate preparation device 203 is provided with a solution A airtight vessel 204 and a solution B airtight vessel 205 as weighing means which weighs the solution A and solution B supplied from the powder A dissolving means 201 and the powder B dissolving means 202, and a liquid storage tank 206 as mixing means which supplies a dialysate to dialysis devices, which are not shown.
A purified water passage 207 which supplies purified water is connected to the above-described powder A dissolving means 201 and powder B dissolving means 202. A solution A supply passage 208 is arranged between the powder A dissolving means 201 and the solution A airtight vessel 204. A solution B supply passage 209 is arranged between the powder B dissolving means 202 and the solution B airtight vessel 205. Solution A discharge passages 210a, 210b are arranged between the solution A airtight vessel 204 and the liquid storage tank 206. Solution B discharge passages 211a, 211b are arranged between the solution B airtight vessel 205 and the liquid storage tank 206.
Furthermore, a dialysate passage 212 is arranged between the liquid storage tank 206 and the dialysis devices. In this dialysate passage 212, there is provided a dialysate circulation passage 212a which branches off from midway and is connected to the liquid storage tank 206, and a twelfth on-off valve SV12 is provided in the dialysate circulation passage 212a.
On the side upstream of the branch position to the above-described dialysate circulation passage 212a in the dialysate passage 212, there are provided a fourth liquid feeding pump P4 which feeds a dialysate to the downstream side and a first concentration sensor CD1 which measures the concentration of a dialysate, and on the side downstream of the branch position, there are provided a second concentration sensor CD2 and a first motor valve MV1.
In this embodiment, under the inflow pressure by a second pump P2 provided in the solution A supply passage 208 and a third pump P3 provided in the solution B supply passage 209, the solution A and the solution B flow into the liquid storage tank 206 from the solution A airtight vessel 204 and the solution B airtight vessel 205, respectively, and these pumps and on-off valves, SV6, SV7, SV10, SV11, which open and close the flow passages from the solution A airtight vessel 204 and the solution B airtight vessel 205 to the liquid storage tank 206 constitute liquid feeding means.

It is ensured that the solution A and the solution B flow from the sides of the above-described liquid storage tank 206 by means of the above-described solution A discharge passages 210a, 210b and the solution B discharge passages 211a, 211b, and are mixed in this liquid storage tank 206, whereby a dialysate is prepared.
A concave portion 206a is formed at the bottom of the liquid storage tank 206, the above-described dialysate passage 212 is connected to the bottom of this concave portion 206a, and the above-described dialysate circulation passage 212a is connected to the bottom of the liquid storage tank 206 where this concave portion 206a is not formed.
The flow rate of the liquid capable of being fed on the side downstream of the branch position of the dialysate circulation passage 212a in the above-described dialysate passage 212, is set at a flow rate smaller than the liquid flow rate of the above-described fourth liquid feeding pump P4, and for this reason, it is ensured that in the dialysate passage 212, part of the dialysate circulates to the liquid storage tank 206 via the dialysate circulation passage 212a. And these constitute stirring means.
Furthermore, a first liquid level sensor LS1 and a second liquid level sensor LS2, each of which detects the stored amount of a dialysate, are provided in the interior of the liquid storage tank 206, and the second liquid level sensor LS2 is provided within the above-described concave portion 206a.
The above-described first liquid level sensor LS1 is adapted to detect that the stored amount of a dialysate in the liquid storage tank 206 has reached a set upper limit or lower, and the above-described second liquid level sensor LS2 is adapted to detect a lack of the remaining amount of a dialysate. It is ensured that when this second liquid level sensor LS2 has detected a lack of the remaining amount of a dialysate, control means gives a prescribed alarm and stops the dialysate preparation device 203.
This liquid storage tank 206 is open to the atmosphere and a filter 251 is provided in a place which communicates with the atmosphere.

Hereinafter, a description will be given of the dialysate preparation method using this dialysate preparation device 203. The operations of the above-described powder A dissolving means 201, powder B dissolving means 202, solution A airtight vessel 204, solution B airtight vessel 205 and fourth to eleventh on-off valves SV4 to SV11 constituting the liquid feeding means are the same as in the second embodiment described above, and hence detailed descriptions thereof are omitted.
In this third embodiment, the solution A and the solution B are simultaneously fed in the same amount from the above-described solution A airtight vessel 204 and solution B airtight vessel 205 to the liquid storage tank 206, and the solution A and the solution B are prepared with set concentrations which are set so that a dialysate can be obtained by mixing the solutions in prescribed proportions without the addition of purified water. Therefore, a dialysate is prepared immediately in the liquid storage tank 206.
It is ensured that in the liquid storage tank 206, the solution A and the solution B flow in and are mixed and the mixed solutions are discharged from the above-described dialysate passage 212 by the action of the fourth liquid feeding pump P4, and it is ensured that the solution A and the solution B are stirred and mixed by closing the first motor valve MV1 and opening the twelfth on-off valve SV12.
And when the dialysate is prepared to an upper limit amount in the liquid storage tank 206, the first motor valve MV1 becomes opened and the dialysate is fed to dialysis devices. It is ensured that also during this liquid feeding, part of the dialysate circulates via the dialysate circulation passage 212a, and it is possible to stir the solution A and the solution B.
In the case where an abnormality in the concentration of a dialysate is detected in the first and second concentration sensors CD, it is ensured that the first motor valve MV1 is closed and the second motor valve MV2 is opened, with the result that the dialysate is discharged via the above-described discharge liquid passage 245.

Also according to the above-described embodiment, because the solution A and the solution B are prepared with the same concentrations as in the first and second embodiments and the solution A and the solution B are supplied simultaneously to the liquid storage tank 206 in proportions of 1:1, a dialysate can be obtained without the addition of purified water and hence it is possible to prepare a dialysate easily.
Also, because it is only necessary for the above-described solution A and solution B to be mixed in the liquid storage tank 206, the configuration becomes simple compared to the conventional art in which two mixing tanks for mixing the undiluted liquid A, undiluted liquid B and purified water and a storage tank have to be installed, and it is possible to miniaturize equipment. Also, the dialysate circulation passage 212a enables a dialysate to be stirred sufficiently.

Incidentally, the above-described reciprocating pump 5 in the first embodiment above may be used as mixing means, such as the accumulator tank 106 in the second embodiment above and the liquid storage tank 206 in the third embodiment, and similarly, the accumulator tank 106 and the liquid storage tank 206 in the second and third embodiment may be used as mixing means of other embodiments.
Furthermore, it is possible to adopt such a configuration that in the second embodiment above, the above-described accumulator tank 106 is provided in a plurality of numbers and the solution A and the solution B are supplied to each accumulator tank 106 by branching the above-described solution A discharge passages 110a, 110b and solution B discharge passages 111a, 111b.
This enables the solution A and the solution B to be sufficiently mixed in the above-described accumulator tanks 106.
And in the first to third embodiments above, whether the solution A and the solution B are prepared with the above-described concentrations may be ascertained by providing concentration sensors of the solution A and the solution B each on the downstream side of the powder A dissolving means 1, 101, 201 and the powder B dissolving means 2, 102, 202.

### Reference Signs List

- 1, 101, 201: Powder A dissolving means
- 2, 102, 202: Powder B dissolving means
- 3, 103, 203: Dialysate preparation device
- 4: Weighing means
- 5: Reciprocating pump
- 104, 204: Solution A airtight vessel
- 105, 205: Solution B airtight vessel
- 106: Accumulator tank
- 205: Liquid storage tank

## Claims

1. A dialysate preparation method for preparing a dialysate which involves preparing a solution A and a solution B by dissolving a dialysis powder A and a dialysis powder B, separately, in purified water and mixing the solution A and the solution B, comprising:
setting beforehand the concentration of the solution A, the concentration of the solution B and prescribed proportions of the solutions so that the dialysate can be prepared by mixing the solution A and the solution B in the prescribed proportions without the addition of purified water;
preparing then a solution A with the set concentration by dissolving the dialysis powder A in purified water and preparing a solution B with the set concentration by dissolving the dialysis powder B in purified water; and
further preparing the dialysate by mixing the solution A with the set concentration and the solution B with the set concentration in the prescribed proportions without the addition of purified water.

2. The dialysate preparation method according to claim 1, wherein so that the dialysate can be obtained by mixing the solution A and the solution B in proportions of 1:1, the concentrations of the solution A and the solution B are set and the solution A and the solution B are mixed in the proportions.

3. A dialysate preparation device, comprising:
weighing means which separately weighs a solution A with a set concentration and a solution B with a set concentration, which are set so that a dialysate can be obtained by mixing the solutions in prescribed proportions without the addition of purified water;
mixing means which prepares a dialysate by mixing the solution A and the solution B, which are weighed by the weighing means; and
liquid feeding means which feeds the solution A and the solution B from the weighing means to the mixing means,
wherein the weighing means weighs the solution A and the solution B in the same number of times so that the prescribed proportions are obtained in the mixing means, and the solution A and the solution B are fed by the liquid feeding means to the mixing means.

4. The dialysate preparation device according to claim 3,
wherein the weighing means is an airtight vessel whose interior is partitioned by a diaphragm into a solution A chamber and a solution B chamber, which have the same capacity,
wherein it is ensured that the solution B which has been weighed is discharged from the solution B chamber while the solution A is being supplied to the solution A chamber and being weighed, and that the solution A which has been weighed is discharged from the solution A chamber while the solution B is being supplied to the solution B chamber and being weighed, and
wherein the liquid feeding means alternately feeds the solution A and the solution B from the solution A chamber and the solution B chamber to the mixing means.

5. The dialysate preparation device according to claim 3,
wherein the weighing means comprises a solution A airtight vessel and a solution B airtight vessel, the interior of both of which is partitioned by a diaphragm into two chambers having the same capacity,
wherein it is ensured that while a solution is being supplied to either of the chambers in each of the airtight vessels, a solution which has been weighed is discharged from the other chamber, and
wherein the liquid feeding means simultaneously feeds the solution A and the solution B from each of the solution A airtight vessel and the solution B airtight vessel to the mixing means.

6. The dialysate preparation device according to claims 3 to 5, further comprising:
powder A dissolving means which prepares a solution A by dissolving a dialysis powder A in purified water; and
powder B dissolving means which prepares a solution B by dissolving a dialysis powder B in purified water,
wherein a solution A with a set concentration and a solution B with a set concentration, which are set so that a dialysate can be obtained by mixing the solutions in the prescribed proportions without the addition of purified water, are prepared by these powder dissolving means.
